# EUROPÄISCHE PATENTSCHRIFT

(11) **EP 2 008 681 B1**
(45) Veröffentlichungstag und Bekanntmachung des Hinweises auf die Patenterteilung: **15.12.2010**
(21) Anmeldenummer: 07111423.5
(22) Anmeldetag: 29.06.2007
(51) Int. Cl.: A61M 25/00, A61M 5/168, A61M 39/22, A61M 39/28, F16K 15/14, F16K 7/02, A61M 39/08

(54) **Vorrichtung zur Verhinderung eines freien Katheterdurchflusses**
Device for preventing free flow in a catheter
Dispositif pour éviter l'écoulement libre d'un liquide dans un cathéter

(43) Veröffentlichungstag der Anmeldung: 31.12.2008
(73) Patentinhaber: F.Hoffmann-La Roche AG, 4070 Basel (CH); Roche Diagnostics GmbH, 68305 Mannheim (DE)
(72) Erfinder: Niklaus, Hanspeter, 4853 Riken (CH); Haenggi, Roger, 4208 Nunningen (CH)
(74) Vertreter: Schwabe - Sandmair - Marx

(56) Entgegenhaltungen:
- EP-A- 0 273 714
- EP-A- 1 466 646
- DE-C- 36 527
- GB-A- 939 324
- US-A- 3 103 335
- US-A- 4 453 696
- US-A- 4 515 589
- US-A- 5 232 193
- US-A1- 2004 087 911
- US-B1- 6 454 742

## Beschreibung

Die vorliegende Erfindung bezieht sich auf eine Vorrichtung zur Verhinderung eines freien Katheterdurchflusses (free flow prevention) und insbesondere auf einen Katheter, welcher so ausgebildet ist, dass ein freier Durchfluss einer Flüssigkeit durch den Katheter aufgrund eines hydrostatischen Druckes der im Katheter geleiteten Flüssigkeit verhindert wird. Des weitem bezieht sich die Erfindung auf ein System mit einem solchen Katheter und einer Verabreichungsvorrichtung, insbesondere einer Infusionspumpe.

Bekannte Infusionssysteme lagern das zu verabreichende Medikament in einem Behältnis, üblicherweise einer Ampulle, in der sich eine Trägerflüssigkeit mit dem darin gelösten Medikament - im folgenden einfach Medikamentflüssigkeit genannt - zwischen einem beweglichen Stopfen und einem Behältnisauslass befindet. Am Behältnisauslass ist ein Katheter mit seinem hinteren Ende angeschlossen. Am vorderen Katheterende sitzt eine Nadel, die zum Verabreichen der Medikamentflüssigkeit in den menschlichen oder tierischen Körper eingeführt wird und dort meist über eine oft mehrtägige Dauer der Verabreichung verbleibt. Befindet sich dabei das Behältnis mit der Medikamentflüssigkeit in einer größeren Höhe als das vordere Katheterende bzw. die Nadel, so besteht bei ausreichender Höhendifferenz zwischen dem Behältnis und dem vorderen Katheterende die Gefahr, dass sich das Behältnis durch die Kraft der Flüssigkeitssäule allmählich selbst entleert.

In der Insulintherapie mit tragbaren Infusionsgeräten, beispielsweise Pumpengeräten, können die verwendeten Katheter Längen von mehr als 1 m aufweisen. Wird das Gerät mit dem Behältnis vertikal über dem Verwender angeordnet, beispielsweise nachts oder beim Duschen, so entsteht ein hydrostatischer Bodendruck von etwa 0. 1 bar, falls neben dem rein statischen Druck aufgrund des Eigengewichts der Medikamentflüssigkeit keine weiteren Effekte, beispielsweise Reibungsverluste, Auslaufeffekte oder Kapilarwirkungen, berücksichtigt werden und für die Medikamentflüssigkeit die Dichte von Wasser angenommen wird.

Zum Verhindern des unerwünschten Auslaufens aufgrund des Drucks der Flüssigkeitssäule könnte die Wandreibung zwischen dem im Behältnis frei gleitend aufgenommenen Stopfen und der Behältniswandung vergrößert werden, was jedoch anderweitige Nachteile mit sich bringen würde. Eine andere Lösung wäre, den Stopfen am Abtriebsglied einer Infusionspumpe zu fixieren, so dass der Stopfen ein Absinken der Flüssigkeitsoberfläche im Behältnis verhindert und dadurch einer Selbstentleerung vorbeugt. Bekannte Systeme verschrauben den Stopfen mit dem Abtriebsglied. Dies erhöht jedoch die Kosten des Geräts. Ferner ist diese Lösung bei vorkonfektionierten Ampullen nicht anwendbar, da der Stopfen für eine Schraubverbindung nicht vorbereitet ist.

Die internationale Anmeldung WO 97/02059 betrifft eine Infusionspumpe mit einem Pumpengehäuse und einem Sicherheitsventil, das alleine durch Schwerkraft verursachte Medikamentenausschüttung verhindern soll. Das Anschlussgehäuse der Pumpe ist an dem Pumpen - gehäuse lösbar befestigt. Ferner ist es mit seinem stromaufwärtigen Ende über einen Katheter und einen Einlasskonnektor mit einem sackförmigen Medikamentenreservoir verbunden.

Die Internationale Patentanmeldung WO 95/16480 offenbart eine Infusionsvorrichtung mit einem Medikamentenbehältnis, einem davon abgehenden Katheter, einer an dem Katheter angeordneten Klemme, einer an den Katheter angeschlossene Pumpe, einem von der Pumpe zum Patienten führenden weiteren Katheter und einem in dem weiteren Katheter angeordneten Sicherheitsventil. Mittels der Klemme für den ersten Katheter und dem Sicherheitsventil soll eine unerwünschte Förderung des Medikamentenfluids aufgrund der Schwerkraft verhindert werden.

Die EP 0 882 466 A2 offenbart eine Vorrichtung zur dosierten Verabreichung, insbesondere Infusion, einer Medikamentflüssigkeit, mit einem Behältnis, aus dem die Medikamentflüssigkeit beim Vorschub eines Stopfens zu ihrer Verabreichung dosiert durch einen Auslass verdrängt wird, und einem am Auslass an das Behältnis angeschlossenen Katheter, dessen vom Auslass abgewandtes vorderes Ende mit einer Verabreichungsnadel verbunden oder verbindbar ist, wobei zwischen dem Auslass und der Verabreichungsnadel in einem Strömungsquerschnitt der Medikamentflüssigkeit ein Ventil angeordnet ist und das Ventil zum Verhindern einer Selbstentleerung einen Durchfluss auf das vordere Ende des Katheters zu nur zulässt, wenn der in diese Richtung wirkende Flüssigkeitsdruck grösser ist als ein auf dem Ventil lastender Druck infolge des Eigengewichts einer Flüssigkeitssäule in der Vorrichtung.

Die EP 1 466 646 A1 betrifft eine Sicherheitseinrichtung für das Anhalten der Strömung einer Flüssigkeit in einem flexiblen Kanal. Die Sicherheitseinrichtung umfasst Backen auf elastischen Armen zum Verschließen der Leitung durch Klemmen des Kanals, wenn der Druck der Flüssigkeit einen vorgegebenen Wert überschreitet.

Die US 6,454,742 B1 betrifft ein Ventil für ein System zum Verabreichen einer Flüssigkeit. Das System umfasst eine Schlauchanordnung mit einem Lumen, das eine Durchleitung zu einer Pumpe aufweist. Das Ventil ist dazu ausgebildet, einen freien Durchfluss einer Flüssigkeit zu verhindern und umfasst einen flexiblen Korpus mit einem zylindrischen Teil, welcher eine Öffnung und einen sich verjüngenden Teil mit abgeschrägten, sich gegenüberliegenden Flächen aufweist. Die Enden der Flächen bilden einen von Seitenwänden umgegebenen Schlitz. Die Öffnung kommuniziert mit dem Schlitz mittels eines durch den Korpus gebildeten Durchgangs zum Durchleiten der Schlauchanordnung. Ein freier Durchfluss durch die Schlauchanordnung wird dadurch verhindert, dass der Schlitz geschlossen ist, wenn die Enden der sich gegenüberliegenden abgeschrägten Flächen in einem entspannten Zustand zusammengepresst sind. Bei Anwendung einer Zugkraft auf die Schlauchanordnung nimmt der innere Durchmesser des Lumens ab, wodurch die Enden der sich gegenüberliegenden abgeschrägten Flächen auseinander gehen, den Schlitz öffnen, und einen Durchfluss durch den Korpus ermöglichen.

Die EP 0 273 714 A2 betrifft einen Pumpenvorsatz für eine Schlauchpumpe oder peristaltischen Pumpe. Der Pumpenvorsatz umfasst ein flexibles äußeres Rohr, einen zusammenklappbaren und ausdehnbaren elastomerischen Schlauch, ein Druckregelventil und ein Ein-Weg-Fluss-Ventil. Das innere Rohr befindet sich in einem Durchgang des Außenrohrs. Das Druckregelventil und Ein-Weg-Fluss-Ventil reagieren auf einen positiven Flüssigkeitsdruck auf einen Einlass zu dem Innenrohr und ermöglichen es der Flüssigkeit in einen Durchgang des inneren Rohrs einzutreten, aus dem die Flüssigkeit durch die Schlauchpumpe gepumpt werden kann. Der Schlauch umfasst einen abgeflachten Einlassbereich der am Einlass endet und einen abgeflachten Auslassbereich der am Auslass endet. Die abgeflachten Bereiche des Innenrohres sind in der Regel geschlossen und öffnen sich infolge eines positiven Flüssigkeitsdrucks. Das Öffnen / Schließen des Einlassbereichs wird durch einen positiven / negativen Druck gesteuert, beispielsweise +/- 0,001 bar, oder +/-0,00025 bar.

Die US 4,515,589 betrifft eine Schlauchpumpe, welche Flüssigkeit zu dem Auslass der Pumpe mittels eines rotierenden Gliedes verdrängt, wobei das rotierende Glied mit einem Schlauch verbunden ist. Der Schlauch besteht aus einem äußeren Schlauch und einem inneren Schlauch mit einem ringförmigen Luftraum. Wenn eine positive Wassersäule über der Wassersäule der Pumpe aufrechterhalten wird, wird sich der innere Schlauch füllen und erweitern. Wenn das Niveau der Flüssigkeit unter der Wassersäule der Pumpe sinkt, fällt der innere Schlauch zusammen, so dass der Schlauch geschlossen und keine Flüssigkeit verdrängt wird und der Ausstoß der Pumpe unterbunden wird so dass eine Einführung von Luft in die Blutbahn des Patienten verhindert wird.

Die US 3,103,335 betrifft eine Klemmvorrichtung für einen Schlauch, durch welchen eine Flüssigkeit, beispielsweise ein Einlaufmittel, durchfließen kann. Die Klemmvorrichtung ist entlang des Schlauchs verschiebbar und positionierbar zum Abstellen des Durchflusses durch den Schlauch mittels Umklappen des Schlauchs.

Die US 4,453,696 betrifft eine Vorrichtung zum Befestigen eines elastischen Schlauchs an einen Rohrstutzen. Um die Sicherheit des elastischen Schlauchs zu gewährleisten ist Rückhalteelement vorgesehen mit einer Öffnung, die einem Durchmesser aufweist, welcher größer als der Durchmesser des Schlauchs ist. Der Durchmesser der Öffnung ist jedoch klein genug zum Festhalten des Schlauchs an den Rohrstutzen.

Die US 939,324 betrifft eine Vorrichtung zum Steuern des Durchflusses durch einen flexiblen Schlauch mittels Verformen des Schlauchs.

Die DE 36527 betrifft eine Verschlussvorrichtung für Schläuche, wobei die Wirkung der Verschlussvorrichtung darauf basiert, dass der durch Ringe oder Haken geführte Schlauch umgeknickt wird.

Die US 5,232,193 betrifft eine Klammer für einen flexiblen Schlauch für ein Infusions- oder Verabreichungsgerät. Die Klammer weist Öffnungen auf, in welche der Schlauch schlangenförmig einführbar ist, so dass er eine Schlaufe bildet. Die Reibung zwischen dem Schlauch und der Oberfläche der Klammer, welche durch die schlangenförmige Konfiguration verursacht wird, ermöglicht es der Klammer an jeder Stelle entlang des Schlauchs positioniert zu werden während der Schlauch in einer Durchflussposition ist.

Es ist eine Aufgabe der Erfindung einen Katheter und ein System bestehend aus einem solchen Katheter und einer Verabreichungsvorrichtung zu schaffen, welche verhindern, dass eine durch den Katheter hindurch abgegebene Medikamentenflüssigkeit oder Substanz unkontrolliert abgegeben wird oder ausläuft.

Diese Aufgabe wird durch den Gegenstände des unabhängigen Anspruchs 1 gelöst. Vorteilhafte Ausführungsformen sind in den abhängigen Ansprüchen definiert.

Ein erfindungsgemäßer Katheter zur Verbindung einer Verabreichungsvorrichtung, wie z.B. einer an sich bekannten Infusionspumpe, mit einer Verabreichungsnadel, welche z.B. in einem so genannten Infusions-Set enthalten sein kann, weist eine Katheterwand auf, welche vorzugsweise als durchgängige einteilige Wand eines vorzugsweise schlauchförmigen oder rohrförmigen. Katheters z.B. aus einem elastischen Material vorgesehen ist. Die Innenseite(n) der Katheterwand begrenzen oder definieren den Durchflussbereich des Katheters, durch welchen eine z.B. von einer Infusionspumpe abgegebene Medikamentenflüssigkeit oder Substanz zu einer Verabreichungsstelle und insbesondere zu einer Verabreichungsnadel gefordert wird. Erfindungsgemäß weist der Katheter mehrere Knickbereiche auf, an welchem zumindest ein Teilstück der Katheterinnenwand an mindestens einem anderen Teilstück der Katheterinnenwand anliegt oder aufliegt, um den Durchfluss durch den Katheter selbsttätig zu blockieren, wobei der Durchfluss wieder ermöglicht und der Katheter geöffnet wird, wenn die in dem Katheter geführte Flüssigkeit einen Druck oberhalb eines vorgegebenen Mindestdruckes von z.B. mehr als 0,1 oder mehr als 0,2 bar aufweist.

Das Vorsehen mehrerer Knickbereiche, welche sich im Normalzustand ohne Anlegen eines vorgebbaren Mindestdruckes der vor dem Katheter geführten Flüssigkeit durch Anbringen der erfindungsgemäßen Klemmstücke, so verformen dass ein Durchfluss durch den Katheter erst bei Überschreiten eines vorgebbaren Mindestdruckes von z.B. mehr als 0,1 bar möglich wird, hat den Vorteil, dass keine zusätzlichen Ventile am Katheterende oder im Katheter vorgesehen werden müssen, um einen freien Durchfluss zu verhindern. Somit kann ein erfindungsgemäßer selbstsperrender Katheter einfach aufgebaut werden und verhindert auch eine Fehlbedienung, da keine zusätzlichen Ventile konnektiert werden müssen und somit auch bei der Verwendung des Katheters nicht vergessen werden können.

Der Katheter weist zwei oder mehr Katheterabschnitte auf, welche z.B. in Längs- oder Durchflussrichtung des Katheters voneinander beabstandet sind, um zwei oder mehr Zonen zu bilden, um einen freien Durchfluss durch den Katheter bei Anliegen eines Druckes unterhalb des vorgegebenen Mindestdruckes zu verhindern. Erfindungsgemäß werden hintereinander zwei oder mehr Zonen ausgebildet innerhalb welcher die Katheterinnenwände aneinander anliegen. Dabei kann die Verhinderung eines freien Durchlaufes bei einem Druck unterhalb des vorgebbaren Mindestdruckes durch ein Zusammenwirken von mehreren hintereinander angeordneten Zonen, in welchen die Katheterwände in Längsrichtung des Katheters voneinander beabstandet an mehreren Abschnitten aneinander anliegen und somit in Längsrichtung mehrere Sperr- oder Sicherungselemente bilden, realisiert werden.

Vorzugsweise liegt der Mindestdruck, welcher anliegen muss, um die Selbstsperrung oder den Selbstverschluss des Katheters wieder zu öffnen, mindestens bei 0,1 bar und vorzugsweise darüber, also im Bereich oberhalb von 0,15 oder 0,2 oder oberhalb von 0,3 bar oder 0,7 bar.

Wen die Ampulle vorgespannt ist, also eine Mindeststoßkraft am Stopfen anliegt, kann es sein, dass der Fluiddruck am Ausgangsbereich der Ampulle bei einem höheren Druck von z.B, 0,5 bar liegt. Folglich sollte der Öffnungsdruck des Katheters bei diesem höheren Druck zuzüglich eines Sicherheitsdrucks von z.B, 0,1 oder 0,2 bar liegen.

Um die dosierte Medikamentverabreichung möglichst wenig zu behindern, aber dennoch ein Auslaufen sicher zu unterbinden, ist der Katheter vorzugsweise so ausgelegt, dass er den Durchfluss in Richtung auf das vordere Ende des Katheters erst zulässt, wenn der Flüssigkeitsdruck in diese Richtung den maximal möglichen Druck der Flüssigkeitssäule, vorzugsweise multipliziert mit einem Sicherheitsfaktor, Übersteigt. Da es sich im vorliegenden Fall um eine Anwendung des Ventils im medizinaltechnischen Bereich handelt, entspricht dieser Sicherheitsfaktor besonders bevorzugt dem Wert 3, Bei einer maximalen Katheterlänge von etwa 1 m und vernachlässigbarer Flüssigkeitssäule im Behältnis beträgt der maximale Flüssigkeitsdruck am freien Ende des Katheters etwa 0,1 bar, so dass der Katheter in diesem Falle so ausgelegt wird, dass er erst öffnet, wenn der Flüssigkeitsdruck 0,3 bar übersteigt. Dies ist auch der Dimensionierungsfall für die bevorzugte Verwendung in einer tragbaren Infusionspumpe.

Die Knickbereiche des Katheters sind selbstschließend oder selbstsperrend unterhalb des oben erwähnten Sperr- oder Mindestdruckes und können somit wirksam verhindern, dass unbeabsichtigt eine Substanz oder Medikamentenflüssigkeit aufgrund des Druckes der Flüssigkeitssäule innerhalb des Katheters durch diesen hindurchfließt.

In einem nicht erfindungsgemäßen Beispiel weist der Katheter Bereiche oder Abschnitte auf, in welchen die Schlauchwandungen gegeneinander vorgespannt sind. Dies kann z.B. dadurch erfolgen, dass nur ein Teilstück in Umfangsrichtung des Katheters in Richtung auf eine andere z.B. gegenüberliegende Katheterinnenwand vorgespannt ist, oder dadurch, dass zwei z.B. einander gegenüberliegende Katheterwandbereiche in Richtung aufeinander vorgespannt sind, um bei fehlendem Druck des in dem Katheter geführten Mediums vorzugsweise aneinander anzuliegen, so dass eine Sperrung des Durchflusses unterhalb des erwähnten einen Durchfluss ermöglichenden Mindestdruckes realisiert wird. Die Vorspannung eines Katheterteilstückes oder eines Bereichs der Katheterwand kann beispielsweise durch die Verwendung eines elastischen Materials realisiert werden, welches an oder in dem Katheter oder als Teil der Katheterwand so vorgesehen ist, dass eine Katheterwand oder mehrere Katheterwände in eine Richtung vorgespannt werden, welche den Durchflussbereich, also eine Öffnung innerhalb des Katheters, verringern und bevorzugt ganz verschließen.

Alternativ oder ergänzend kann auch durch ein externes Element, wie z.B. eine Klammer oder Klemmvorrichtung, welche z.B. als U-förmiges Federelement ausgebildet ist, eine externe Kraft erzeugt werden, welche auf die Katheteraußenwand oder Katheteraußenwände wirkt, um den Katheter zwischen dem oder durch das Klammer- oder Federelement zusammenzudrücken. Eine durch den Katheter hindurchtretende Flüssigkeit kann bei Anliegen des vorgebbaren Mindestdruckes, z.B. erzeugt durch eine Infusionspumpe, das externe Element so weit auseinanderdrücken, dass ein in Abhängigkeit von dem anliegenden Druck begrenzter oder auch ungehinderter Durchfluss durch den Katheter ermöglicht wird.

In einem weiteren nicht erfindungsgemäßen Beispiel kann der Katheter auch durch einen Fertigungsprozess oder durch ein externes Element, wie z.B. ein Klemmelement, so umgeformt werden, dass der Katheter im Querschnitt nicht ringförmig ist, wie bekannte Katheter, sondern eine andere Form aufweist, welche zur Realisierung einer Durchflusssperrung vorteilhaft ist, wie z.B. die Form eines Flachschlauches, bei welcher die Wandungshälften aneinander anliegen und somit einen Durchfluss bei einem geringen Druck unterbinden können.

Erfindungsgemäß ist der Katheter gebogen oder geknickt ausgeführt wobei diese gebogene oder geknickte Form durch das den Katheter in einer gebogenen oder geknickten Form haltende Klemmstück wie in Anspruch 1 definiert realisiert wird.

Gemäß einem weiteren Aspekt bezieht sich die Erfindung auf ein System mit einem wie oben beschriebenen vorrichtung aus Klammer und Katheter und einer Verabreichungsvorrichtung, insbesondere einer Infusionspumpe zur dosierten Verabreichung einer Medikamentftüssigkeit, bei der die Medikamentflüssigkeit in einem Behältnis enthalten ist, aus dem sie durch Vorschub eines im Behältnis beweglich aufgenommenen Stopfens auf einen Behältnisauslass zu in dosierter Weise zu ihrer Verabreichung verdrängt wird. Unmittelbar am Auslass des Behältnisses, wo ein Auslassstück bzw, -stutzen zum Anschliessen eines Katheters vorhanden ist, kann ein wie oben beschriebener Katheter mit seinem hinteren Ende angeschlossen werden. Üblicherweise handelt es sich um einen schlauchförmigen Katheter. Die Verwendung eines starren Katheters mit elastischen Teilstücken wäre jedoch ebenso möglich. Das vordere, freie Ende des Katheters ist mit einer Nadel zur Verabreichung des Medikaments verbunden oder mit solch einer Nadel verbindbar. Unter einer Verabreichung wird sowohl eine Infusion als auch eine Injektion und auch eine Kombination beider Verabreichungsarten verstanden. Insbesondere zielt die Erfindung auf die Verwendung bei Infusionsvorrichtungen bzw. -geräten ab. Besonders bevorzugt handelt es sich um tragbare Pumpengeräte in der Insulinbehandlung.

Die Vorrichtung aus Klammer und Katheter ist zwischen dem Behältnisauslass und der Nadel zum Verabreichen des Medikaments in einem Strömungsquerschnitt der Medikamentflüssigkeit angeordnet.

Obwohl die Vorrichtung grundsätzlich an einer beliebigen Stelle zwischen den Behältnisauslass bzw. der Infusionspumpe und der Verabreichungsnadel sitzen kann, wird sie vorzugsweise so nah wie möglich beim Auslass des Behältnisses oder so nah wie möglich an der Infusionspumpe angeordnet.

Ein Verfahren zum Verhindern eines freien Durchflusses durch einen Katheter, wobei mindestens ein Teilbereich des Katheters oder die Katheterwand den Durchfluss eines Mediums unterhalb eines vorgegebenen Mindestdruckes verhindert und wobei der Katheter aufgedrückt wird, um den Durchfluss zu ermöglichen, wenn das Medium den Mindestdruck übersteigt, ist ebenfalls offenbart.

Die Erfindung wird nachfolgend anhand bevorzugter Ausführungsformen erläutert. Es zeigen:
- Figur 1: eine Draufsicht auf ein Katheterteilstück gemäß einen nicht erfindungsgemäßen Beispiel mit einer Klammer;
- Figur 1A: eine Schnittsicht entlang der Linie A-A in Figur 1;
- Figur 1B: eine Schnittansicht entlang der Linie B-B in Figur 1;
- Figur 2: ein zweites nicht erfindungsgemäßes Beispiel eines selbstsperrenden Katheters mit zwei Sperrebereichen;
- Figur 2A: eine Querschnittsansicht entlang der Linie A-A in Figur 2;
- Figur 3: eine schematische Darstellung zur Erläuterung des Wirkprinzips der Erfindung;
- Figur 4: eine Ausführungsform der Vorrichtung aus Klammer und Katheter gemäß der Erfindung mit mehreren hintereinander angeordneten Knickbereichen; und
- Figur 5: ein weiteres nicht erfindungsgemäßes Beispiel eines selbstsperrenden Katheters mit einem separaten Druckelement,

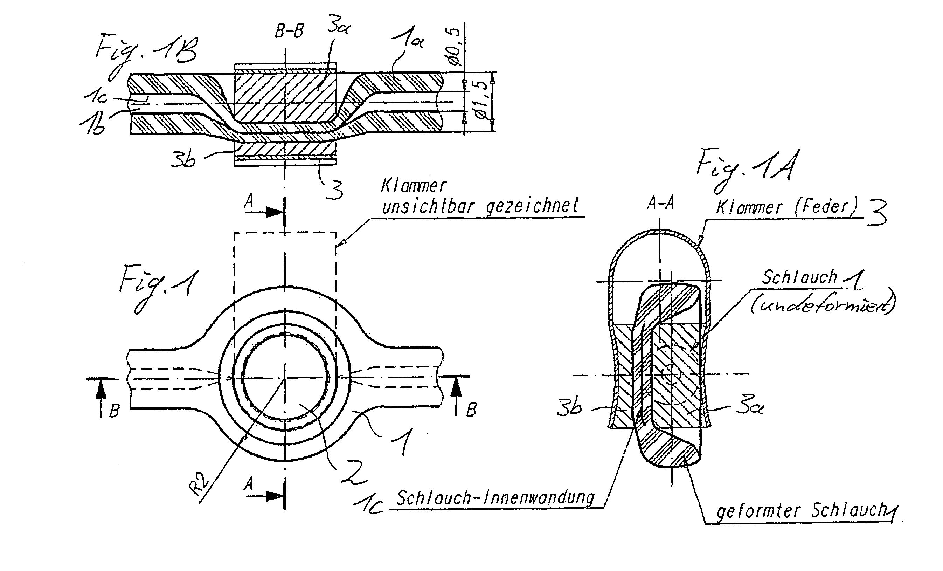

Figur 1 zeigt ein nicht erfindungsgemäßes Beispiel eines an einem Katheter 1 ausgebildeten Sperrventils 2, welches direkt an bzw, in dem Katheterschlauch bevorzugt in der Nähe der Abgangsstelle der Pumpe (nicht gezeigt) ausgebildet ist. Das Sperrventil 2 ist so ausgestaltet, dass es bei einem Schlauchinnendruck bzw. Druck des in dem Schlauch 1 geführten Mediums von 0,1 bar oder weniger sperrt und sich bei höheren Drücken öffnet und einen Durchfluss durch den Durchflussbereich 1b des Katheters 1 erlaubt.

Das gezeigte Beispiel des Katheters 1 kann z.B. durch eine Katheter- bzw. Schlauchumformung im Bereich des Ventils 2 mittels eines Wärmeumformverfahrens erhalten werden. Der ring- oder rohrförmige Querschnitt des Katheters 1 wird dabei im Bereich des Ventils 2 in einen Flachschlauch umgeformt, wie in der Schnittansicht in Figur 1A gezeigt, so dass die Wandungshälften 1a des Katheters 1 aneinander anliegen und somit einen Durchfluss bei einem geringen anliegenden Innendruck unterbinden können. Steigt der Druck des in dem Katheter 1 geführten Mediums oberhalb des vorgegebenen Mindestdruckes von z.B. 0,1 bar, wird die Katheterinnenwandung 1c aufgestoßen und das Ventil 2 geöffnet, so dass ein Durchfluss durch den Katheter 1 möglich ist.

Wie insbesondere aus den Figuren 1A und 1B ersichtlich ist, kann das Ventil 2 mit einer Klammer 3, welche als U-förmiges Federelement ausgebildet ist, mit Andruckstücken 3a und 3b ausgestattet sein. Die Klammer 3 ist um den Katheter 1 herum angeordnet und kann über ihre Klemmkraft die Katheterhälften und damit die Katheterinnenwände 1c gegeneinander vorspannen. Mittels der Klammer 3 kann eine definierte Sperrkraft bzw. ein definierter Sperrdruck eingestellt werden. Je größer die Kraft ist, mit welcher die Klammer 3 die Katheterwände 1c aneinanderdrückt, umso größer ist der Mindestdruck des Mediums, welcher anliegen muss, um einen Durchfluss durch den Katheter 1 zu ermöglichen.

Wird ein solcher Katheter 1 mit einem oder mehreren z.B. in Längsrichtung des Katheters 1 hintereinander angeordneten Ventilen 2 an eine Verabreichungsvorrichtung, wie z,B, eine Infusionspumpe, angeschlossen, ist der Katheter 1 mit einem Reservoir 4 für eine zu verabreichende Substanz bzw. ein Medium verbunden. Üblicherweise wird von einer Infusionspumpe ein Stopfen 5 in das Reservoir 4 bzw. eine Ampulle 4 eingeschoben, so dass es in der Ampulle 4 zu einer Erhöhung des Innendruckes kommt und eine in der Ampulle 4 enthaltene Substanz aus einer Abgabeöffnung 4a der Ampulle 4 abgegeben wird. Dies führt in dem angeschlossenen Katheter 1 zu einem Druckanstieg, welcher dafür sorgt, dass die gegeneinander vorgespannten Katheterwandungen 1a aufgestoßen werden und dadurch eine gewünschte Menge der Substanz, wie z.B. Insulin, an einen Patienten abgegeben werden kann. Sobald die gewünschte Menge der Substanz verabreicht ist und z.B. der Stopfen 5 des Reservoirs 4 wieder in Ruhe ist, schließt sich das Ventil 2 aufgrund der Kraft der Klammer 3, welche den Katheter 1 wieder zusammendrückt, von selbst, Über die Vorspannkraft der Klammer 3 wird somit der Öffnungsdruck für das Ventil 2 eingestellt oder definiert.

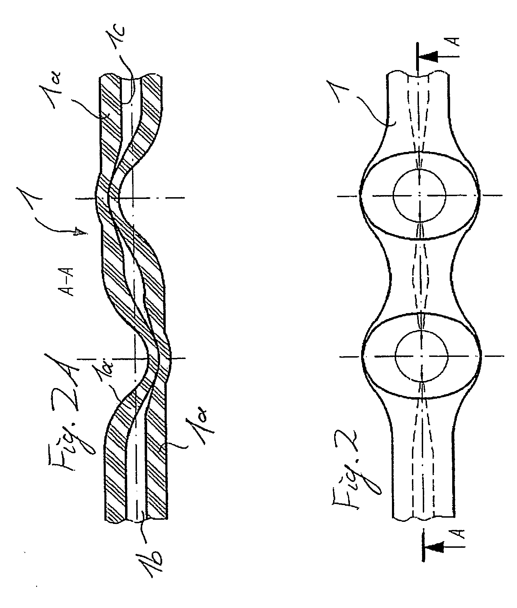

Figur 2 zeigt ein weiteres Beispiel eines nicht erfindungsgemäßen Katheters 1 mit zwei Ventilen 2, Die beiden Ventile 2 sind in zwei in Längsrichtung des Katheters 1 voneinander beabstandeten Zonen angeordnet und weisen jeweils gegeneinander vorgespannte Katheterwandungen 1a auf. Dabei ist eine in Figur 2A links gezeigte Katheterwandung 1a einer ersten Seite in Richtung auf die zweite Seite (nach unten) so vorgespannt, dass die Katheterinnenwände 1a aneinander anliegen und einen Durchfluss eines Mediums verhindern, wenn nicht ein vorgegebener Mindestdruck anliegt. Im Bereich des rechts gezeigten zweiten Ventils ist die Vorspannung relativ zum ersten Ventil umgekehrt, das heißt die gegenüberliegende zweite Seite ist in Richtung auf die erste Seite so vorgespannt, dass die gewünschte Ventilsperrwirkung auftritt.

Jede Vorspannung einer Katheterwand 1 a kann dabei als eine Blende angesehen werden, Bei einer Serienschaltung mehrerer Blenden wird der Gesamtdruckunterschied in Stufendruckunterschiede unterteilt, so dass der Druckabfall pro Ventilzone reduziert werden kann. Eine Reduzierung des Gesamtdruckunterschiedes ist vorteilhaft, da sich dadurch die Schließkraft pro Ventilzone verringern lässt und so die Funktionssicherheit des aus mehreren Ventilzonen gebildeten Gesamtventils erhöht werden kann.

Gemäß einen weiteren Beispiel kann eine Ventilfunktion mit einem Katheter 1 dadurch erzielt werden, dass der Katheter 1 mit einem definierten Wegkraft-Element (Feder) in einen Knickbereich gebracht wird. Bei ansteigendem Druck, z.B. zwischen 0,5 und 0,8 bar, befreit sich der Katheter 1 aus dem Knickbereich und gibt so den Durchfluss frei,

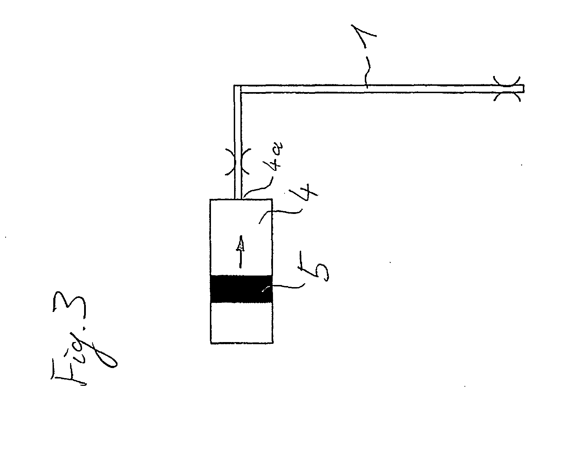

Figur 3 zeigt schematisch eine Ampulle 4 mit einem darin in Pfeilrichtung verschiebbaren Stopfen 5, mit welchem eine in der Ampulle 4 enthaltene Substanz aus einer Abgabeöffnung 4a an den an die Ampulle 4 angeschlossenen Katheter 1 abgegeben werden kann. Würde der Höhenunterschied zwischen dem unteren und dem oberen Ende des Katheters 1 beispielsweise einen Meter betragen, so würde am oberen Ende des Katheters 1 ein Unterdruck von 0,1 bar anliegen, welcher dazu führen könnte, dass unbeabsichtigt aus der Ampulle 4 allein aufgrund des hydrostatischen Drucks, welcher durch die Flüssigkeitssäule in dem Katheter 1 erzeugt wird, eine Substanz aus der Ampulle 4 unkontrolliert abgegeben wird.

Es ist daher vorteilhaft, ein Ventil im oberen Katheterbereich anzuordnen, da ein Unterdruck von 0,1 bar im Bezug zu einem relativen Aussendruck von 0 bar zu einem selbstständigen Zusammenpressen der Katheterwände 1a führt. Der Unterdruck im oberen Katheterabschnitt kann daher bei elastischen Katheterwänden 1a derart ausgenutzt werden, dass im Falle einer hydrostatischen Druckverteilung im Katheter eine automatische Schließung des Ventils erfolgt.

Wird der oben beschriebene Katheter 1 verwendet, so kann verhindert werden, dass aus der Ampulle 4 unbeabsichtigt eine Substanz durch den Katheter 1 abgegeben wird.

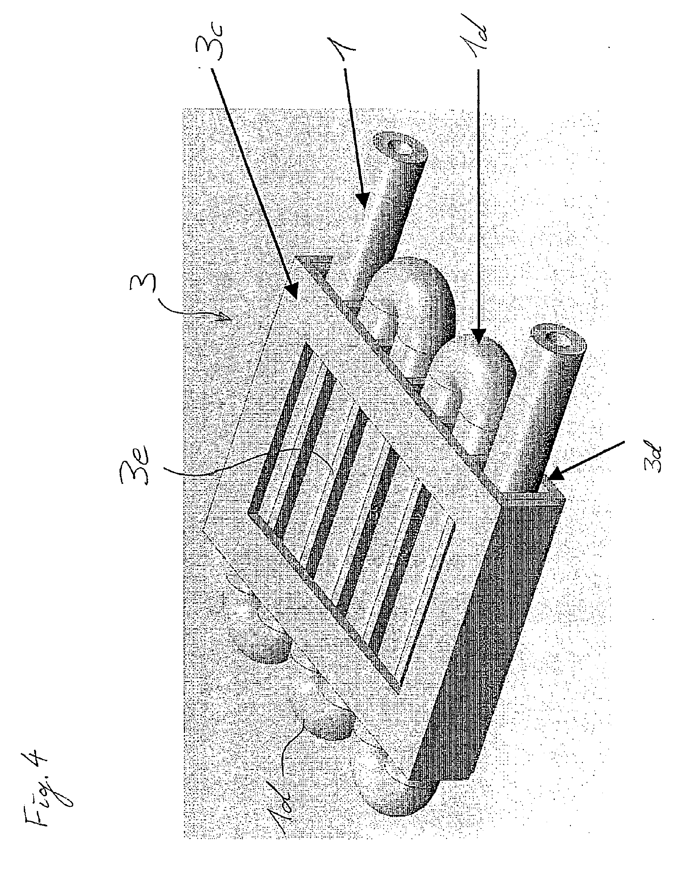

Erfindungsgemäß werden mehrere Knickbereiche 1d hintereinander angeordnet werden, wie in Figur 4 gezeigt. Dabei wird ein Katheter oder Schlauch 1 in einem Klemmstück oder einer Klammer 3 aufgenommen, welche aus einem Klemmstück-Oberteil 3c und einem Klemmstück-Unterteil 3d besteht, die miteinander verbunden sein können. Der Katheter 1 wird so in die z.B. durch mehrere parallel zueinander liegende Trennwände 3e gebildeten Katheterführungen eingelegt, dass der Katheter 1 z.B. geschlängelt oder schlangeförmig oder gewunden in dem Klemmstück 3 gehalten wird, Dabei liegen gerade Bereiche des Katheters 1 parallel zueinander durch die Wandstücke 3e getrennt, wobei jeweils im Bereich des Übergangs des Katheters 1 von einer durch zwei benachbarte Wandstücke 3e gebildeten Führung zur nächsten Führung ein Knickbereich 1d vorliegt. Figur 4 zeigt den Zustand mit geöffnetem Katheter 1, bei welchem die Knickbereiche 1d aufgrund eines anliegenden Druckes von z.B. größer als 0,7 bar geöffnet sind und so einen Durchfluss eines Mediums durch den Katheter 1 hindurch ermöglichen. Liegt ein Druck unterhalb von 0,7 bar an, so ist der Katheter 1 im Bereich der Knicke 1d so verformt, dass ein hindurchtreten eines Mediums durch den Katheter 1 verhindert wird.

Das Klemmstück 3 kann z.B. starr ausgebildet sein, so dass sich bei einem Druckanstieg des in dem Katheter 1 geführten Mediums auf über 0,7 bar allein durch die Verformung des Katheters 1 eine Durchflussmöglichkeit ergibt. Alternativ kann das Klemmstück 3 auch elastisch oder federnd ausgebildet sein, so dass sich das Klemmstück 3 bei einem Druckanstieg des in dem Katheter 1 geführten Mediums verformt oder ausdehnt und so den Durchfluss für die in dem Katheter 1 gefühlte Flüssigkeit freigibt.

Zum einfacheren Einlegen des Katheters 1 ist das Klemmstück 3 aus zwei Teilstücken 3c und 3d ausgebildet, welche aufeinander aufgesetzt oder miteinander verbunden werden können, nachdem der Katheter 1 in das Klemmstück 3d auf die in Figur 4 gezeigte Art so eingelegt wurde, dass der Katheter 1 mehrere hintereinander liegende Knickbereiche 1d bevorzugt auf zwei gegenüberliegenden Seiten des Klemmstückes 3 aufweist.

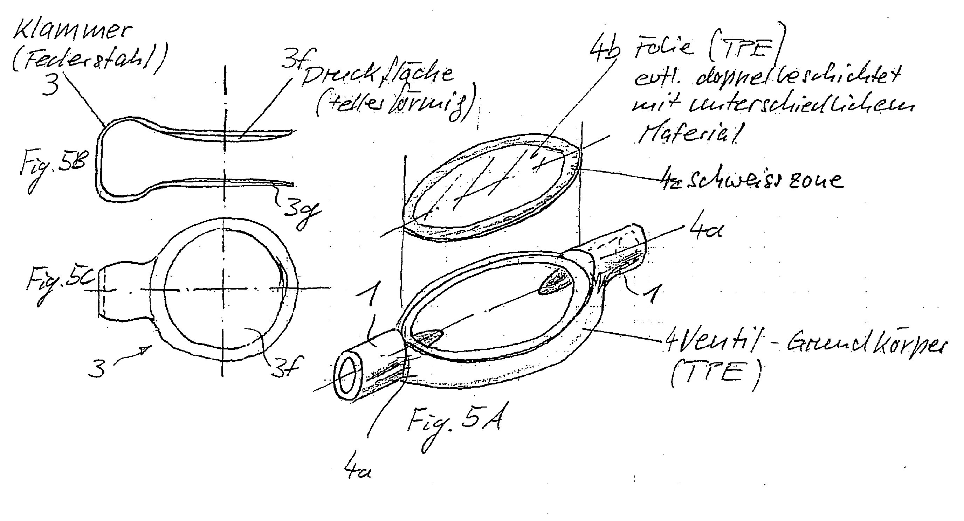

Figur 5A zeigt ein weiteres nicht erfindungsgemäßes Beispiel eines Sperrmechanismus für einen Katheter 1 Dabei kann ein Katheter-Sperrventil sowohl in den Katheter 1 integriert werden bzw. integriert sein, als auch als separates Ventilstück ausgebildet sein, welches zwischen zwei Katheterstücke 1 eingesetzt werden kann. In den in Figur 5A gezeigten Beispiel ist ein aus einem thermoplastischen Elastomer (TBE) gefertigter Ventil-Grundkörper 4 ersichtlich, welcher scheibenförmig oder tellerförmig und bevorzugt flach ausgebildet und zwischen zwei Katheterstücken 1 eingesetzt wurde, wobei der Ventil-Grundkörper 4 an die Katheterstücke 1 im Bereich einer Schweißzone 4a angesetzt und mit den Katheterstücken 1 verbunden oder verschweißt wurde, um so ein integrierter Bestandteil des Katheters zu sein. Auf den Ventil-Grundkörper 4 ist ein Verschluss- oder Abdeckelement 4b in Gestalt einer ebenfalls aus einem thermoplastischen Elastomer geformten Folie aufgesetzt, welche doppelbeschichtet und aus einem anderen Material gebildet sein kann, als der Ventil-Grundkörper 4. Die Folie 4b wird mit dem Ventil-Grundkörper 4 im Bereiche einer Schweißzone 4c verbunden.

Auf den mit der Folie 4b verbundenen Ventil-Grundkörper 4 wird ähnlich wie in den zu Figur 1 beschriebenen Beispiel eine aus Federstahl gebildete gebogene Klammer 3 aufgeschoben oder aufgesetzt, welche zwei einander gegenüberliegende Druckflächen .3f und 3g aufweist, so dass z.B. die obere Druckfläche 3f auf die Folie 4b und die untere Fläche 3g auf die Unterseite des Ventil-Grundkörpers 4 drücken kann. Figur 5C ist eine Draufsicht auf die in Figur 5B in einer Seitenansicht gezeigte Klammer 3.

Liegt an dem in dem Katheter 1 transportierten Medium ein Druck von z.B. kleiner als 0,7 bar an, so wird die Folie 4b durch die Klammer 3 so in den Ventil-Grundkörper 4 eingedrückt bzw. dieser zusammen mit der Folie 4b so zusammengedrückt, dass kein Durchfluss eines Mediums stattfinden kann. Steigt der Druck des Mediums auf über 0,7 bar an, so wird die Klammer 3 durch den Druck des Mediums auseinander gedrückt, d.h. die Druckflächen 3f und 3g werden voneinander weggedrückt, um einen Durchfluss eines Mediums zu ermöglichen.

Durch die Auslegung der Klammer 3 und insbesondere die Herstellung, Verformung und Materialkonstanten oder Bearbeitung kann der Sperrdruck des in Figur 5 gezeigten Ventils verändert werden, so dass sich das Ventil auch bei einem kleineren Druck von z.B. 0,1 oder 0,2 bar oder bei einem größeren Druck von z.B. 1 bar oder darüber öffnet.

## Patentansprüche

1. Vorrichtung zur Verhinderung eines freien Katheterdurchflusses umfassend
- einen Katheter (1) mit einer Katheterwand (1a), einem durch die Katheterwand (1a) begrenzten Durchflussbereich (1b) zur Verbindung einer Verabreichungsvorrichtung mit einer Verabreichungsnadel, und
- eine Klammer (3) zum Aufnehmen des Katheters (1),
**gekennzeichnet dadurch, dass**
- der Katheter (1) durch die Klammer (3), umfassend ein Oberteil (3c) und ein Unterteil (3d), so aufgenommen wird, dass
- mehrere gerade Bereiche des Katheters (1) parallel zueinander liegen,
- jeweils im Bereich des Übergangs zwischen zwei geraden Bereichen Knickbereiche (1d) vorliegen, und
- mehrere Knickbereiche (1d) hintereinander angeordnet sind, und
- wobei in den Knickbereichen zumindest ein Teilstück einer Katheterinnenwand (1c) an mindestens einem anderen Teilstück einer Katheterinnenwand (1c) anliegt, um den Durchfluss eines Mediums durch den Katheter (1) zu blockieren, wobei der Durchfluss geöffnet wird, wenn das Medium einen Druck aufweist, welcher oberhalb eines vorgegebenen Sperrdruckes liegt.

2. Vorrichtung nach Anspruch 1, wobei der Sperrdruck bei 0,1 oder 0,2 oder 0,3 bar oder 0,7 bar oder darüber liegt.

3. System mit einer Vorrichtung nach einem der vorhergehenden Ansprüche und einer Verabreichungsvorrichtung, wie z.B. einer Infusionspumpe, wobei ein Ende des Katheters (1) mit der Verabreichungsvorrichtung verbunden ist.

4. System nach dem vorhergehenden Anspruch mit einer Nadel und/oder einem Infusions-Set, welche/s mit dem Katheterende verbunden ist, welches dem Katheterende gegenüberliegt, das mit der Verabreichungsvorrichtung verbunden ist.

5. System nach einem der zwei vorhergehenden Ansprüche, wobei die Klammer (3) im Bereich oder nahe an der Verabreichungsvorrichtung angeordnet ist.

## Claims

1. A device for preventing a free flow through a catheter, comprising:
- a catheter (1), comprising: a catheter wall (1a); a flow region (1b), limited by the catheter wall (1a), for connecting an administering device to an administering needle; and
- a clip (3) for accommodating the catheter (1),
- **characterised in that**
- the clip (3), comprising an upper part (3c) and a lower part (3d), accommodates the catheter (1) such that
- a number of linear regions of the catheter (1) are parallel to each other,
- sharply bent regions (1d) are provided in each region which transitions between two linear regions, and
- a number of sharply bent regions (1d) are arranged successively, and
- wherein in the sharply bent regions, at least one partial piece of an inner wall (1c) of the catheter abuts at least one other partial piece of an inner wall (1c) of the catheter, in order to block the flow of a medium through the catheter (1), wherein the flow is opened when the medium exhibits a pressure above a predetermined blocking pressure.

2. The catheter according to claim 1, wherein the blocking pressure is around 0.1 bar or 0.2 bar or 0.3 bar or 0.7 bar or above.

3. A system comprising a device according to any one of the preceding claims and an administering device, such as for example an infusion pump, wherein one end of the catheter (1) is connected to the administering device.

4. The system according to the preceding claim, comprising a needle and/or an infusion set which is connected to the end of the catheter opposite the end of the catheter which is connected to the administering device.

5. The system according to any one of the preceding two claims, wherein the clip (3) is arranged in the region of or near to the administering device.

## Revendications

1. Dispositif pour empêcher un écoulement libre à travers un cathéter, comportant :
- un cathéter (1) ayant une paroi de cathéter (1a), une zone d'écoulement (1b) délimitée par la paroi de cathéter (1a) pour relier un dispositif d'administration à une aiguille d'administration, et
- un dispositif de serrage (3) pour recevoir le cathéter (1),
**caractérisé en ce que**
- le cathéter (1) est reçu par le dispositif de serrage (3) comportant une partie supérieure (3c) et une partie inférieure (3d) de telle sorte que
- plusieurs zones rectilignes du cathéter (1) sont parallèles les unes aux autres,
- des zones coudées (1d) se situent respectivement dans la zone de la transition entre deux zones rectilignes, et
- plusieurs zones coudées (1d) sont disposées les unes derrière les autres, et
- dans lequel, dans les zones coudées, au moins une portion partielle d'une paroi intérieure de cathéter (1c) se situe au moins sur une autre portion partielle d'une paroi intérieure de cathéter (1c) afin de bloquer l'écoulement d'un fluide à travers le cathéter (1), l'écoulement étant ouvert lorsque le fluide a une pression qui est supérieure à une pression de blocage prédéterminée.

2. Dispositif selon la revendication 1, dans lequel la pression de blocage est de 0,1 ou 0,2 ou 0,3 ou 0,7 bar ou supérieure.

3. Système comportant un dispositif selon l'une quelconque des revendications précédentes et un dispositif d'administration tel que, par exemple, une pompe à perfusion dans lequel une extrémité du cathéter (1) est reliée au dispositif d'administration.

4. Système selon la revendication précédente, comportant une aiguille et/ou un ensemble de perfusion relié à l'extrémité de cathéter située en face de l'extrémité de cathéter qui est reliée au dispositif d'administration.

5. Système selon l'une quelconque des deux revendications précédentes, dans lequel le dispositif de serrage (3) est agencé dans la zone ou à proximité du dispositif d'administration.
